# EUROPEAN PATENT APPLICATION

(11) **EP 3 699 575 A1**
(43) Date of publication of application: **26.08.2020**
(21) Application number: 18856201.1
(22) Date of filing: 13.09.2018
(51) Int. Cl.: G01N 21/25, G01N 29/02, G01N 23/00, G01N 33/18

(54) **SENSOR HAVING ENHANCED DETECTION CAPABILITY**

(30) Priority: 13.09.2017 KR 20170117211
(71) Applicant: Giparang Co., Ltd., Seoul 08826 (KR)
(72) Inventor: LEE, Sang Don, Guri-si Gyeonggi-do 11948 (KR)
(74) Representative: Boden, Keith McMurray
(86) International application number: PCT/KR2018/010758
(87) International publication number: WO 2019/054773

(57) **Abstract**

A sensor according to the present embodiment comprises: a light provision unit comprising a light-emitting device for providing an optical stimulus, and a first controllable power source for providing driving power to the light-emitting device; a light detection unit comprising a light-receiving device for detecting an optical response to the optical stimulus and then outputting same as an electric signal, and a second controllable power source for providing driving power to the light-receiving device; and a control unit for controlling the driving powers provided by the first controllable power source and the second controllable power source.

## Description

### [Technical Field]

The present invention relates to a sensor.

### [Background Art]

Sensors detect whether targets are present and concentrations of the targets. General sensors detect targets within limits of detection (LODs) but typically cannot detect targets which are located outside the LODs.

### [Disclosure]

### [Technical Problem]

Various efforts have been made to expand a detection range within which a target can be detected. Particularly, a system using positive feedback to expand a limit of detection (LOD) and the like has been developed. According to such a technology, a range within which a target can be detected is dramatically expanded. However, since an operational amplifier (OP-AMP) having a positive feedback structure directly drives a light-emitting diode (LED), an expensive OP-AMP having enhanced voltage driving capability should be used, and power consumed by the OP-AMP is increased.

The present embodiment is for solving such a problem. That is, the present embodiment is directed to providing a sensor which uses relatively cheap elements, decreases power consumption, but has an enlarged limit of detection (LOD).

### [Technical Solution]

One aspect of the present invention provides a sensor including a stimulation source unit having an actuator configured to provide a stimulus and a first controllable power source configured to provide driving power to the actuator, a detection unit having a detection device configured to detect a response to the stimulus and output an electric signal corresponding the response and a second controllable power source configured to provide driving power to the detection device, and a control unit configured to control the driving power provided by the first controllable power source and the driving power provided by the second controllable power source according to a digital code.

### [Advantageous Effects]

A sensor according to the present embodiment has an advantage in that a limit of detection (LOD) is expanded as compared to a conventional technology.

### [Description of Drawings]

FIG. 1 is a schematic block diagram illustrating a sensor according to a present embodiment.
FIG. 2 is a schematic block diagram illustrating a sensor which provides an optical stimulus and detects an optical response.
FIGS. 3A and 3B are schematic block diagrams illustrating a detection unit.
FIGS. 4A and 4B are schematic block diagrams illustrating a control unit.
FIG. 5 is a schematic block diagram illustrating a sensor according to another embodiment.
FIGS. 6A and 6B are schematic graphs each illustrating a current-voltage curve of a light-receiving element of a sensor.
FIG. 7A is a schematic graph illustrating a current-voltage curve of a light-receiving element in an operation of a sensor according to another embodiment, and FIG. 7B is a graph illustrating an example in which a concentration of a target material included in a medium is detected by the sensor according to the present embodiment.
FIG. 8 is a graph illustrating an example in which a result of measuring an optical response using the sensor according to the present embodiment.
FIG. 9 is a graph illustrating a rate of an optical response of 1 nM of bovine serum albumin (BSA) to an optical response of deionized water.
FIG. 10 is a graph illustrating a result of detecting a target using an embodiment of a sensor in which a negative resistance range is not generated.

### [Modes of the Invention]

Since descriptions related to the present invention are provided as exemplary embodiments illustrating structures and functions thereof, it should not be interpreted that the scope of the present invention is limited to the embodiments described in the specification. That is, since the embodiments are susceptible to various modifications and alternative forms, it should be understood that the scope of the invention covers equivalents falling within the spirit of the present invention.

Meanwhile, terms described in the specification should be understood as follows.

The singular forms "a," "an," and "the" are intended to include the plural forms as well, unless the context clearly indicates otherwise. It will be further understood that the terms "comprises," "comprising," "includes," and/or "including," when used herein, specify the presence of stated features, integers, steps, operations, elements, components, and/or groups thereof but do not preclude the presence or addition of one or more other features, integers, steps, operations, elements, components, and/or groups thereof.

Sizes, heights, thicknesses, and the like of components illustrated in the accompanying drawings referred to in described embodiments of the present invention are intentionally exaggerated for the sake of convenience in the description and ease of understanding and are not proportionally enlarged or reduced. In addition, certain components illustrated in the drawings may be intentionally illustrated in an enlarged manner and the other components may be intentionally illustrated in a reduced manner.

Unless otherwise defined, all terms (including technical and scientific terms) used herein are to be interpreted as is customary in the art to which this invention belongs. It should be further understood that terms in common usage should also be interpreted as is customary in the relevant art and not in an idealized or overly formal sense unless expressly so defined herein.

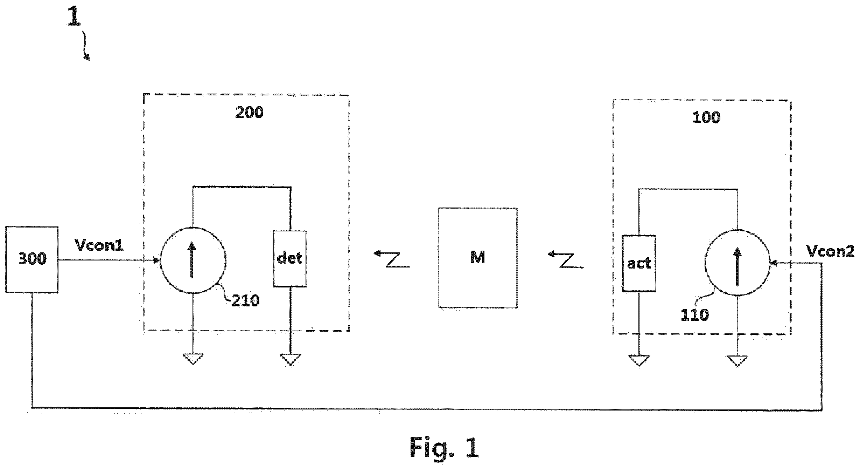

Hereinafter, a sensor 1 according to a present embodiment will be described with reference to the accompanying drawing. FIG. 1 is a schematic block diagram illustrating a sensor according to the present embodiment. Referring to FIG. 1, the sensor 1 according to the present embodiment includes a stimulation source unit 100 including an actuator act configured to provide a stimulus to a medium and a first controllable power source 110 configured to provide driving power to the actuator act, a detection unit 200 including a detection device det configured to detect a response to the stimulus and a second controllable power source 210 configured to provide driving power to the detection device det, and a control unit 300 configured to control power provided from any one of the first controllable power source 110 and the second controllable power source 210.

The actuator act may receive the driving power from the first controllable power source 110 and provide the stimulus to a medium M including a target. As one embodiment, the actuator act may be an optical actuator, and the optical actuator receives driving power and applies an optical stimulus to the medium M including a detection target. Hereinafter, an actuator which provides any one among ultraviolet light, visible light, infrared light, and laser light is defined as an optical actuator. As an example, the optical actuator may be formed as a light-emitting diode (LED), a laser diode (LD), or the like configured to receive a bias to provide light.

The LED may emit light in a visible, ultraviolet, or infrared wavelength band, and the LD may emit laser light in a specific wavelength band within a wavelength band ranging from 270 nm to 3330 nm. The optical actuator may be provided to emit light in a suitable wavelength band according to properties of a material to be detected by a sensing system.

As another embodiment, the actuator act may apply a non-optical stimulus such as sonic waves, ultrasonic waves, radio frequency (RF) waves, radiation, a magnetic field, an electric field, or the like, and when a bias is applied to a non-optical actuator, the non-optical actuator applies a non-optical stimulus to the medium M including a detection material. Hereinafter, an actuator configured to provide any one among the sonic wave, the ultrasonic wave, the RF wave, the radiation, the magnetic field, and the electric field electric field is defined as a non-optical actuator. The non-optical actuator may be formed as an apparatus which may apply the non-optical stimulus such as sonic waves, ultrasonic waves, RF waves, radiation, a magnetic field, or an electric field.

The optical actuator applies the optical stimulus, the non-optical actuator applies the non-optical stimulus, and each actuator performs the same function of providing a stimulus having an extent corresponding to a bias when the bias is applied thereto.

The detection unit 200 includes the second controllable power source 210 and the detection device det configured to receive driving power from the second controllable power source 210 and detect a response of the medium M to the stimulus. As one embodiment, the detection device det may be an optical detection device configured to detect an optical response of the medium M to output an electrical signal corresponding thereto. As an example, the detection device may be a photo diode, a photo transistor, or a photo multiplier tube (PMT), receive driving power from the second controllable power source 210, detect an optical response of the medium, and output a corresponding electrical signal.

As another embodiment, the detection device det may be a non-optical detection device configured to detect a non-optical response of a medium and output an electrical signal. As an example, the non-optical detection device may be a sensor configured to detect physical changes of a medium and a target when sonic waves or ultrasonic waves are provided to the medium M, or a sensor configured to detect an electrical, magnetic, or physical change of any one of a medium and a target when an electric field or a magnetic field is provided to the medium M. In addition, as an example, the non-optical detection device may be a sensor configured to detect physical changes or radiation transmittances of a medium and a target when radiation is provided to the medium M.

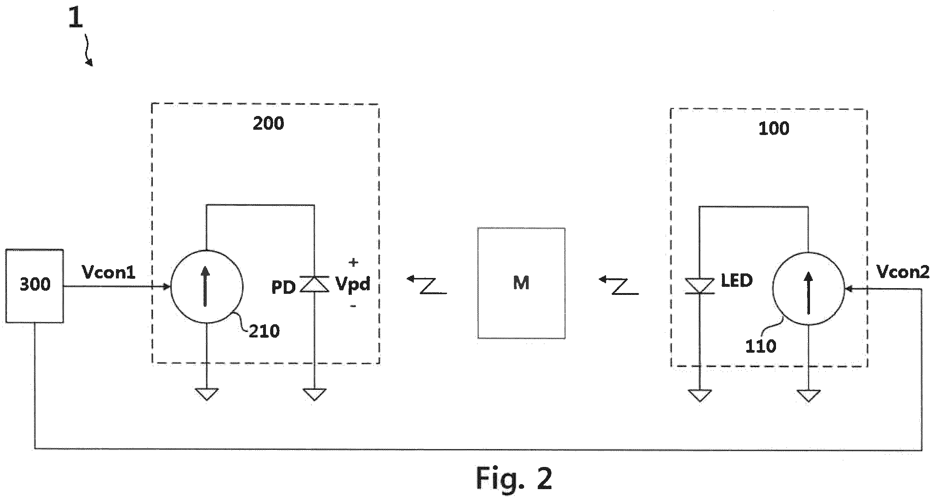

Hereinafter, for the sake of convenience of description, an embodiment in which an optical actuator applies an optical stimulus to a medium M and a detection unit 200 detects an optical response to the optical stimulus will be mainly described as illustrated in FIG. 2. However, the embodiment does not exclude an embodiment in which radiation which is a non-optical stimulus is provided to a medium and an optical response is detected or an electric field, which is a non-optical stimulus, is provided to a medium and an electric field attenuation rate, which is a non-optical response, is detected. The embodiment is only to easily describe the present invention and not to limit the scope of the present invention.

Referring to FIG. 2, a bias current is supplied to a light-emitting element LED from the first controllable power source 110 to provide an optical stimulus to the medium M, and an intensity of the optical stimulus may correspond to the provided bias current. In addition, the light-emitting element LED may have a threshold value and may not provide the optical stimulus when a voltage or current less than the threshold value is applied thereto. As another example, the light-emitting element may emit laser light in a specific wavelength band within a wavelength band ranging from 270 nm to 3330 nm.

The optical stimulus is provided to a detection target. As an example, the target may be included in the medium M. When the optical stimulus is provided to the target, the target optically responds. As an example, the optical response may be one or more among light absorption, light condensation, light scattering, light reflection, and light transmission. As an example of the optical response, bovine serum albumin (BSA) has a property of absorbing light in a wavelength band of 270 to 280 nm. Accordingly, when laser light having a wavelength of 275 nm is emitted to a medium including the BSA, the BSA optically responds to the applied optical stimulus to absorb the applied light.

However, the above described example is only for describing the present invention, and a detection material, an optical stimulus applied to the detection material, and an optical response, which occurs according to the detection material, to the optical stimulus may be changed.

A light-receiving element PD detects an optical response and outputs a corresponding electrical signal. As one embodiment, a bias current may be supplied to the light-receiving element PD from the second controllable power source 210 configured to provide driving power.

The sensor according to the present embodiment sweeps a bias current being provided to the light-emitting element LED and a bias current being provided to the light-receiving element PD to detect an optical response provided by the medium. As will be described below, when the bias currents sweep, a photodetection device 300 may have a negative resistance characteristic.

The sensor may further include a voltage clamping element 220 connected in parallel to the light-receiving element PD. Due to a property of a photodiode which is reversely biased and driven, the sensor may receive a sufficiently high current from the photodiode PD when operating near a breakdown voltage. However, since a breakdown phenomenon may occur due to a reverse bias, there may be a reliability problem when the photodiode operates.

In order to provide a sufficiently high current with a low reverse voltage provided to the light-receiving element PD, the voltage clamping element 220, which prevents and clamps a voltage value applied to the light-receiving element from increasing to a voltage greater than or equal to a target voltage, is connected in parallel to the photodiode. The voltage clamping element prevents the voltage value applied to both ends thereof from increasing to a clamping voltage value or more. Accordingly, the voltage clamping element having a predetermined clamping voltage is connected in parallel to the photodiode to prevent a reverse voltage from being applied to an extent in which a reliability problem of the photodiode occurs.

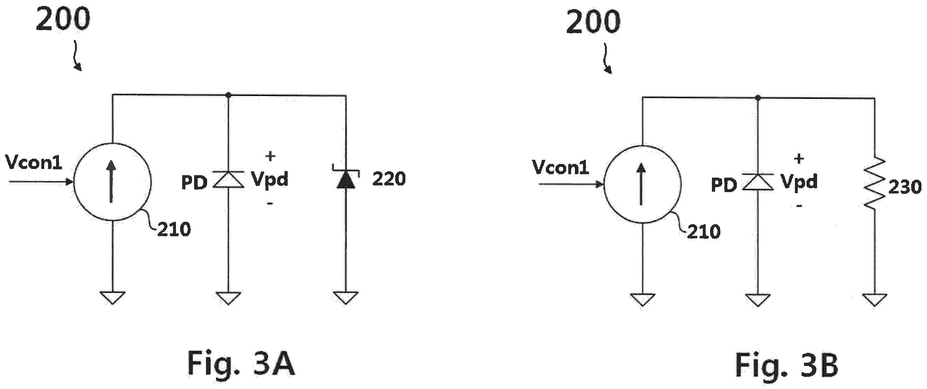

As one embodiment, as illustrated in FIG. 3A, a voltage clamping element may be implemented using a Zener diode using a Zener breakdown phenomenon. When a voltage greater than or equal to a Zener breakdown voltage is applied to the Zener diode in a reverse direction, the Zener breakdown phenomenon may occur such that a reverse current is not blocked but flows and a voltage is clamped to prevent a voltage greater than or equal to the Zener breakdown voltage from being applied to both ends of the Zener diode. Accordingly, when the Zener diode is connected in parallel to the photodiode, an advantage of providing a sufficient current even without applying a voltage greater than or equal to a voltage causing a reliability problem of the photodiode is provided.

As illustrated in FIG. 3B, the sensor may further include a resistor 230 connected in parallel to the light-receiving element PD. As an example, when an optical response of a target is detected and a voltage applied to both ends of the light-receiving element sweeps from zero to several tens of volts, a current value sweeps about several tens of nanoamperes, an average resistance value in the corresponding section is calculated as about several to several tens of gigaohms.

When two resistors in which a resistance value of any one resistor is greater than a resistance value of the other resistor are connected in parallel, an equivalent resistance value of the resistors connected in parallel is approximated to the lower resistance value of the two resistance values. Accordingly, when the light-receiving element PD having a large resistance value is connected in parallel to the resistor having a resistance value less than the large resistance value, an equivalent resistance value of a circuit in which the light-receiving element PD is connected in parallel to the resistor may be approximated to the lower resistance value so that an equivalent resistance may be generated in a range in which measurement may be easily performed without using an expansive measurement apparatus.

As one embodiment, a resistance value of a resistor 230 connected in parallel to a light-receiving element PD may be adjusted according to a material and a concentration thereof to be detected by a sensing system according to the present embodiment. As another example, a resistance value of the resistor 230 connected in parallel to the light-receiving element PD may be adjusted such that an equivalent resistance value of the light-receiving element PD is within a measurement range of a measurement apparatus configured to measure the equivalent resistance value.

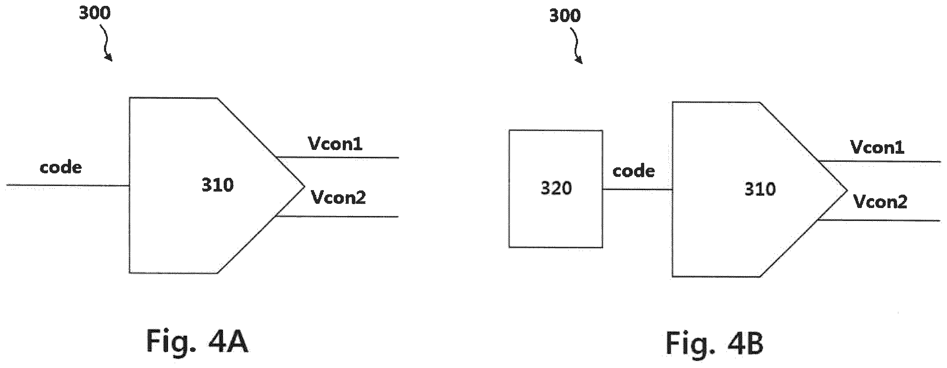

FIGS. 4A and 4B are schematic views illustrating embodiments of the control unit 300. In the embodiment illustrated in FIG. 4A, the control unit 300 includes a digital-to-analog converter 310. The digital-to-analog converter 310 receives a digital code from the outside of the sensor and provides corresponding control voltages Vcon1 and Vcon2 to the first controllable power source 110 and the second controllable power source 210 to control the first controllable power source 110 and the second controllable power source 210. In the embodiment illustrated in FIG. 4B, the control unit 300 further incudes a memory 320 which stores a code. The digital-to-analog converter 310 receives a digital code from the memory 320 and provides control voltages Vcon1 and Vcon2 corresponding to the code to control the first controllable power source 110 and the second controllable power source 210.

As one embodiment, as illustrated in FIG. 4, the control unit 300 may include the digital-to-analog converter 310. The digital-to-analog converter 310 receives a control code from the outside and generates the corresponding control voltages Vcon1 and Vcon2. The generated control voltages Vcon1 and Vcon2 are provided to the first controllable power source 110 and the second controllable power source 210. Although not illustrated in the drawings, the digital-to-analog converter 310 may provide control voltages to a plurality of light provision units 100a, 100b, to lOOn.

The control unit 300 provides the control voltages Vcon1 and Vcon2 to the first controllable power source 110 and the second controllable power source 210 so as to sweep values of bias currents provided by the first controllable power source 110 and the second controllable power source 210 according to the digital code provided from the outside. As an example, the control voltage Vcon1 and the control voltage Vcon2 provided by the control unit 300 are provided to the first controllable power source 110 and the second controllable power source 210 so as to increase the bias currents provided to the light-emitting element LED and the light-receiving element PD.

As an example, the control unit 300 provides the control voltage Vcon1 and the control voltage Vcon2 such that a current provided to the light-emitting diode LED by the first controllable power source 110 is linearly increased and a current provided to the light-receiving element PD by the second controllable power source 210 is linearly or non-linearly increased.

As another example, the control unit 300 provides the control voltage Vcon1 and the control voltage Vcon2 such that a current provided to the light-emitting diode LED by the first controllable power source 110 is non-linearly increased and a current provided to the light-receiving element PD by the second controllable power source 210 is linearly or non-linearly increased.

Although a case in which a current provided to the light-emitting diode LED by the first controllable power source 110 and a current provided to the light-receiving element PD by the second controllable power source 210 are increased has been exemplarily illustrated, conversely, a case in which a current provided to the light-emitting diode LED by the first controllable power source 110 and a current provided to the light-receiving element PD by the second controllable power source 210 are decreased may also be similarly applied.

In the embodiments illustrated in FIGS. 2 and 4, the first controllable power source 110 and the second controllable power source 210 are illustrated as controllable current sources, but these are only embodiments, and the first controllable power source 110 and the second controllable power source 210 may be controllable voltage sources.

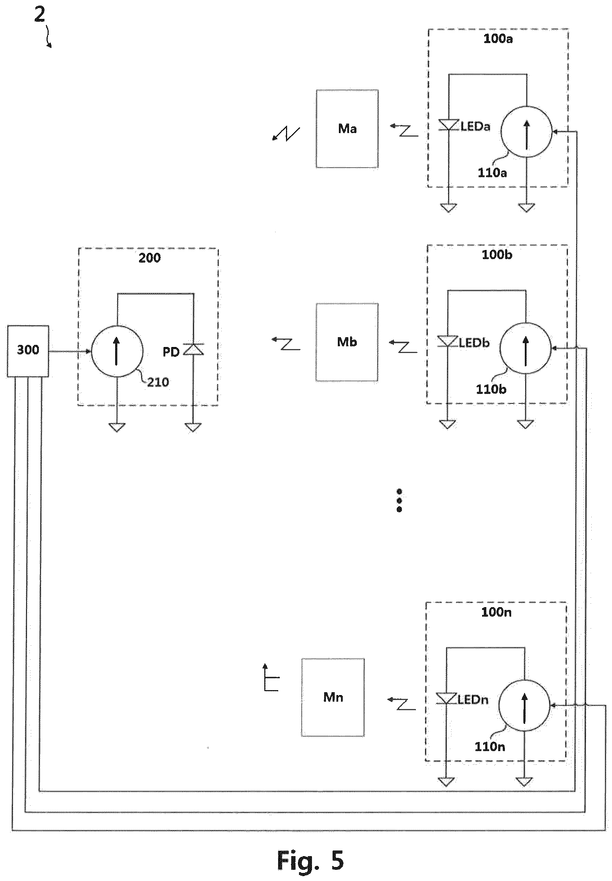

FIG. 5 is a schematic block diagram illustrating a sensor 2 according to an embodiment. Referring to FIG. 5, the sensor 2 may include a plurality of light provision units 100a, 100b, to 100n. As one embodiment, a plurality of light provision units 100a, 100b, to lOOn may provide different optical stimuli. As an example, the light provision unit 100a may provide an ultraviolet light stimulus, the light provision unit 100b may provide an optical stimulus of a blue light wavelength band, and the light provision unit lOOn may provide an optical stimulus of a red light wavelength band. Media to which the optical stimuli are provided by the plurality of light provision units 100a, 100b, to lOOn may be one medium including a target, a plurality of media including the same targets, a plurality of media including the same targets of which concentrations are different, one medium including different targets, or a plurality of media including different targets.

As another embodiment, a plurality of light provision units 100a, 100b, to lOOn may provide optical stimuli to different media. As an example, the light provision unit 100a may provide an optical stimulus to a medium Ma including a target of which a concentration is a first concentration, the light provision unit 100b may provide an optical stimulus to a medium Mb including the target of which a concentration is a second concentration, and the light provision unit 100n may provide an optical stimulus to a medium Mn including the target of which a concentration is a third concentration.

According to another embodiment which is not illustrated in the drawing, a sensor may include one or more light provision units and one or more non-optical actuators to provide stimuli and detect responses of media to the stimuli using one or more optical detection devices and one or more non-optical detection devices.

According to still another embodiment which is not illustrated in the drawing, a sensor may include a plurality of detection units. As an example, the sensor may include a plurality of detection units capable of detecting optical responses and non-optical responses to provided stimuli. As another example, the sensor may include a plurality of detection units configured to detect a response to a stimulus in an infrared wavelength band, a response to the stimulus in a visible wavelength band, and a response to the stimulus in an ultraviolet wavelength band.

In a case in which an increasing rate of a bias provided to a detection device det by a second controllable power source 210 is less than an increasing rate of a bias provided to an actuator act by the first controllable power source 110, the detection device has a negative resistance characteristic. As the increasing rate of the bias provided to the actuator act by the first controllable power source 110 is gradually increased, the negative resistance characteristic may not be generated.

In addition, the negative resistance characteristic may be generated or not generated according to characteristics, a concentration, and optical properties or non-optical properties of a target included in a medium. As an example, even in a case in which biases are set to be provided to the detection device det and the actuator act such that a current-voltage characteristic of the detection device has a negative resistance characteristic, the negative resistance characteristic may not be generated according to characteristics, a concentration, and optical properties of a medium. In addition, even in a case in which biases are set to be provided to the detection device det and the actuator act such that a current-voltage characteristic of the detection device does not have a negative resistance characteristic, the negative resistance characteristic may be generated according to characteristics, a concentration, and optical properties of a medium.

As an example, optical properties of a target included in a medium may be properties such as light dispersion, light absorption, light scattering, and light condensation.

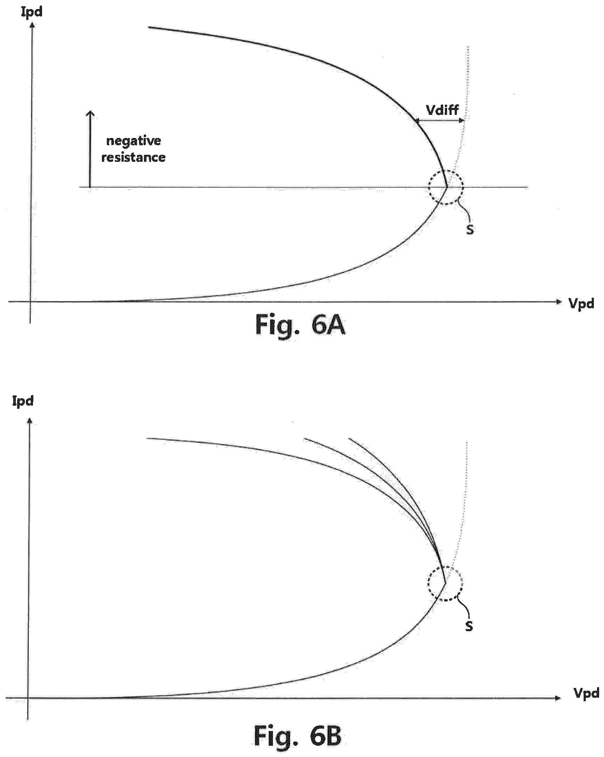

FIGS. 6A and 6B are schematic graphs illustrating current-voltage curves of a light-receiving element according to embodiments of a sensor operation. A sensor operation according to the present embodiments will be described with reference to FIGS. 6A and 6B. In the embodiments illustrated in FIGS. 6A and 6B, the light-receiving element PD is a photodiode, and the light-emitting element LED is a light-emitting diode.

Referring to FIG. 6A, in a state in which the light-emitting element LED is turned off because a bias current provided to the light-emitting element LED by the first controllable power source 110 does not reach a threshold value of the light-emitting element LED, as a reverse bias is increased due to a bias current provided to the light-receiving element PD by the second controllable power source 210, a reverse saturation current of the light-receiving element is increased. This corresponds to a current-voltage characteristic of a conventional light-receiving element PD.

When a voltage provided to the light-emitting element LED by the first controllable power source 110 increases to be greater than a threshold voltage of the light-emitting element LED, the light-emitting element LED is turned on and provides an optical stimulus to the medium M including a target. The target receives the optical stimulus and optically responds thereto, and the light-receiving element PD detects the optical response and provides the optical response in the type of a current.

Since a current component flowing through the light-receiving element PD includes a reverse saturation current component and also further includes a current component due to the optical stimulus, a voltage for generating a reverse saturation current is decreased, and thus a voltage difference Vdiff is generated with respect to a current-voltage characteristic of the light-receiving element PD.

In addition, when the bias current provided to the light-emitting element LED is gradually increased, since an intensity of the optical stimulus provided by the light-emitting element is increased, a current component which is output by the light-receiving element after the light-receiving element detects the optical response is also increased. In order to compensate for this, a voltage applied to both ends of a photodetection device should be gradually decreased. Accordingly, a voltage Vpd applied to both ends of the light-receiving element PD is changed to be decreased. As a result, the light-receiving element PD has a negative resistance characteristic after a time point S at which the light-emitting element LED is turned on.

FIG. 6B is a graph illustrating a current-voltage curve of the light-receiving element PD in a case in which the sensor according to the present embodiment detects included targets of which concentrations are different from each other. Referring to FIG. 6B, a current-voltage characteristic of the light-receiving element PD is the same as the conventional light-receiving element before the time point S at which the light-emitting element LED included in the sensor is turned on. However, when the optical stimulus is provided to the targets after the time point S at which the light-emitting element LED is turned on, different intensities of optical responses and different intensities of responses are detected according to the concentrations of the targets. Accordingly, the light-receiving element PD generates different current-voltage curves corresponding to the optical responses.

Accordingly, after the time point at which the light-emitting element LED is turned on, even when the same amount of current flows through the light-receiving element PD, voltages Vpd applied to both ends of the light-receiving element PD may be different according to concentrations of the targets included in a medium. The concentrations of the targets may be measured by detecting the voltages Vpd. As another example, even in a case in which the voltages Vpd applied to both ends of the light-receiving element PD is the same, currents flowing through the light-receiving element PD may be different, and concentrations of targets may be measured by detecting the currents. As still another example, a concentration of a target may be measured by detecting a current value and a voltage value from which the light-receiving element PD starts to have a negative resistance characteristic.

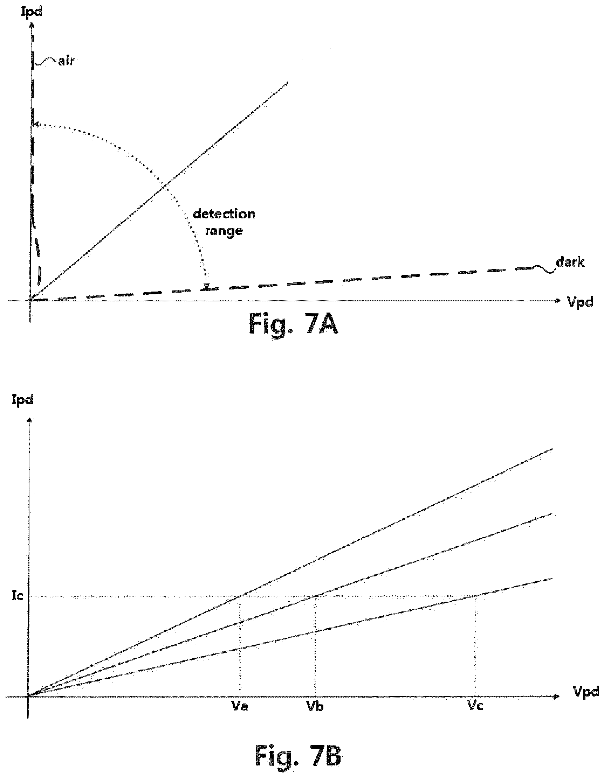

FIG. 7A is a schematic graph illustrating a current-voltage curve of a light-receiving element in an operation of the sensor according to another embodiment. In embodiments illustrated in FIGS. 7A and 7B, the light-receiving element PD is a photodiode and the light-emitting element LED is a light-emitting diode. During an initial operation, in a case in which the light-emitting element is turned on like a case in which a voltage greater than a threshold value of the actuator is provided, a negative resistance region may not be generated.

A current-voltage curve of the light-receiving element PD may have a shape shown in FIG. 7A. As an example, a line marked as "dark" shows a case in which an optical response is not transmitted to the light-receiving element at all, and in this case, a current Ipd flowing through the light-receiving element PD includes a reverse saturation current component due to a voltage Vpd applied to both ends of the light-receiving element PD. A line marked as "air" shows a case in which the light-receiving element detects an entire optical stimulus provided by the light-emitting element in a state in which there are no media, and it can be seen that a negative resistance is generated. In a case in which the light-receiving element detects an optical response of a medium including a target, a current-voltage relation is generated within a detection range illustrated with a dotted line between "dark" and "air" in FIG. 7A, and the target can be detected.

FIG. 7B is a graph illustrating an example in which a concentration of a target material included in a medium is detected by the sensor according to the present embodiment. Referring to FIG. 7B, optical responses may be different from each other according to concentrations of media, and electrical signals output by the light-receiving element after the light-receiving element detects the optical responses are different from each other as illustrated in FIG. 7B. In a case in which the sensor according to the present embodiment is used to detect a concentration of a target and/or a concentration, after the same current Itest is provided to the light-receiving element, concentrations of targets may be measured by detecting voltages Va, Vb, and Vc formed on both ends of the light-receiving element. According to another embodiment which is not illustrated in the drawings, after the same voltage is provided to the light-receiving element, concentrations of targets may be measured by detecting currents output by the light-receiving element.

Even by using the embodiments illustrated in FIGS. 7A and 7B, a target can be detected with high sensitivity. As an example, in a case in which an optical response is detected in a state in which a bias provided to the detection device is fixed, a current-voltage line marked as "dark" is only moved parallel to an extent corresponding to a current detected on an optical response. However, according to the present embodiment, since a bias provided to the detection device is changed, a slope of the current-voltage line is changed within the detection range, and thus an output is sensitively changed even when an optical response is small. Accordingly, there is an advantage in that a target can be detected with high sensitivity even using the present embodiment.

That is, in a case in which the sensor according to a conventional technology does not have a negative resistance, the sensor has a current-voltage relation having a slope which is similar to a slope in a dark state, and as light is provided to the detection device, a current-voltage relation line having a corresponding slope is moved parallel along a voltage axis and/or a current axis in a state in which the slope is maintained. Accordingly, changes in values of a pair of current-voltage coordinates generated due to the provided light are not large.

However, according to the present embodiment, since biases provided to the detection device and the actuator are changed, the slope of the current-voltage relation is changed within a range illustrated as the detection range in FIG. 7A. Since an electrical response occurs largely in the detection device even when a medium includes a target of which a concentration is the same, the target can be detected more sensitively, and a limit of target detection can expand.

### Experimental Example

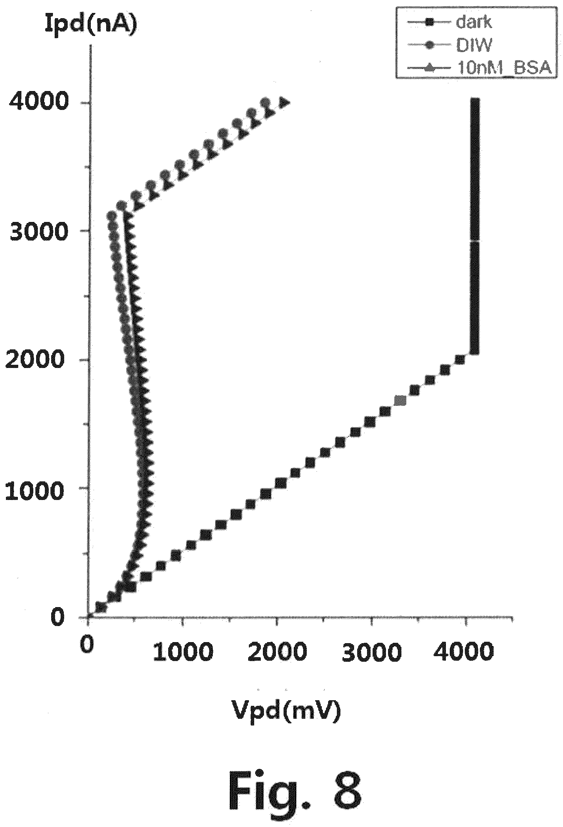

Hereinafter, an experimental example of the sensor according to the present embodiment will be described with reference to the accompanying drawings. FIG. 8 is a graph illustrating an example in which a result of measuring an optical response using the sensor according to the present embodiment and shows an experimental result of measuring an optical response in a dark state in which the light-emitting element is not turned on, and thus, the light-receiving element cannot detect an optical response at all, measuring an optical response of deionized water (DIW), and measuring an optical response of 10 nM of bovine serum albumin (BSA).

Referring to FIG. 8, in the dark state, the light-emitting element LED was not turned on. Accordingly, it may be seen that the light-receiving element PD did not detect an optical response and a negative resistance characteristic was not generated. (A voltage Vpd saturated at 4000 mV is caused by an input dynamic range of the digital-to-analog converter using in the present experiment.)

However, in a state in which the light-emitting element was operated, negative resistance ranges were generated after a time point at which a voltage Vpd of 600 mV was provided in both cases in which the DIW was measured and the 10 nM of the BSA, that was a target, was measured. Even when the same voltages were provided to the light-receiving element as an input, in the case in which the 10 nM of BSA, that was the target, was measured, a current which was less than a current in the case in which the DIW was measured was output.

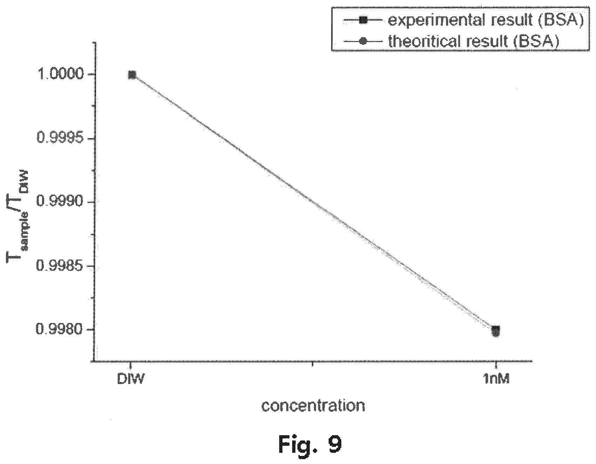

FIG. 9 is a graph illustrating a rate of an optical response of 1 nM of BSA to an optical response of DIW. Referring to FIG. 9, it was seen that the rate of the optical response of the 1 nM of the BSA to the optical response of the DIW is approximated to about 0.9980. Therefore, according to the sensor of the present embodiment, the DIW and the BSA of which a concentration was 1 nM could be definitively distinguished.

Since the sensor according to the present embodiment may detect a target of which a concentration is 1 nM and has a limit of detection which is lower than a lowest limit of detection ranging from 100 nM to 50 nM of the conventional sensor, it may be seen that performance of the sensor is improved.

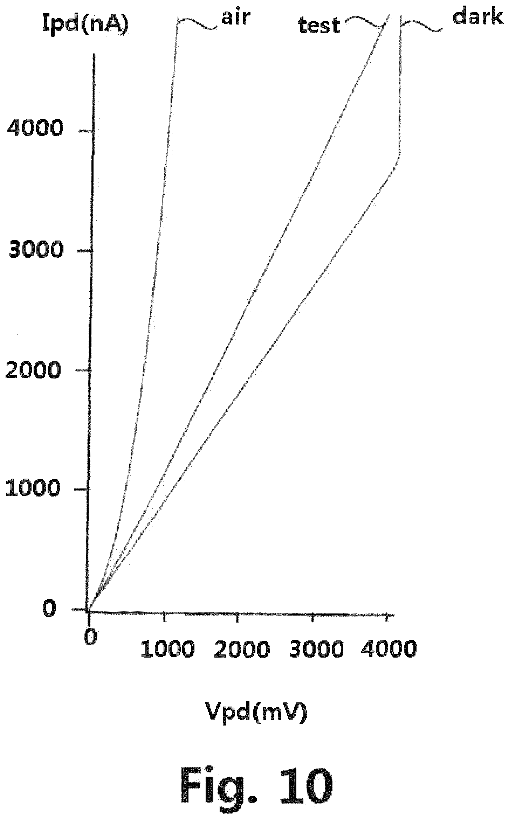

FIG. 10 is a graph illustrating a result of detecting a target using an embodiment of a sensor in which a negative resistance range is not generated. In FIG. 10, "dark" shows a current-voltage relation of a light-receiving element in a case in which the light-receiving element did not detect an optical response at all (a voltage Vpd saturated at 4000 mV is caused by an input dynamic range of a digital-to-analog converter using in the present experiment.), and a line marked as "air" shows a case in which the light-receiving element detects an entire optical stimulus provided by a light-emitting element without a medium.

Test shows a current-voltage relation when the sensor according to the present embodiment detects phenol of which a concentration is 100 µM. When a target within a detection range is detected, a current-voltage relation of the light-receiving element is substantially linearly approximated, and a concentration of the target may be detected using the current-voltage relation.

The present invention has been described with reference to the example embodiments illustrated in the drawings so as to promoting an understanding of the present invention, but these are only examples. It will be understood by those skilled in the art that various modifications and equivalent other example embodiments may be made. Therefore, the scope of the present invention is defined by the appended claims.

### [Industrial Applicability]

Has been described in the [Modes of the Invention].

## Claims

1. A sensor comprising:
a stimulation source unit including an actuator configured to provide a stimulus and a first controllable power source configured to provide driving power to the actuator;
a detection unit including a detection device configured to detect a response to the stimulus and output an electric signal corresponding the response and a second controllable power source configured to provide driving power to the detection device; and
a control unit configured to control the driving power provided by the first controllable power source and the second controllable power source according to a digital code.

2. The sensor of claim 1, wherein the actuator includes an optical actuator configured to provide an optical stimulus.

3. The sensor of claim 2, wherein the optical stimulus comprises one or more selected from the group consisting of infrared light, visible light, ultraviolet light, and laser light.

4. The sensor of claim 1, wherein the actuator includes a non-optical actuator configured to provide a non-optical stimulus.

5. The sensor of claim 4, wherein the non-optical stimulus comprises one or more selected from the group consisting of a sonic wave, an ultrasonic wave, a radio frequency (RF) wave, radiation, a magnetic field, and an electric field.

6. The sensor of claim 1, wherein:
the response to the stimulus is an optical response; and
the detection device includes an optical detection device configured to detect the optical response.

7. The sensor of claim 6, wherein the optical detection device incudes any one selected from the group consisting of a photodiode, a photo transistor, and a photomultiplier tube (PMT).

8. The sensor of claim 6, wherein the optical response includes any one among light transmission, light absorption, light scattering, and fluorescence.

9. The sensor of claim 1, wherein:
the response to the stimulus is a non-optical response; and
the detection device includes a non-optical detection device configured to detect the non-optical response.

10. The sensor of claim 1, wherein:
the stimulus is provided to a target which is a detection target; and
the response is a response of the target to the stimulus.

11. The sensor of claim 1, wherein:
the first controllable power source provides a bias current to the actuator;
the second controllable power source provides a bias current to the detection device; and
the control unit controls the bias current provided by the first controllable power source and the bias current provided by the second controllable power source.

12. The sensor of claim 1, wherein the control unit includes a digital-to-analog converter configured to generate an electrical signal corresponding to the code.

13. The sensor of claim 12, wherein the control unit further includes a memory which stores the code.

14. The sensor of claim 1, wherein the sensor includes a plurality of stimulation source units.

15. The sensor of claim 1, wherein the sensor includes a plurality of detection units.

16. The sensor of claim 1, wherein the sensor detects any one or more selected from the group consisting of a current and a voltage of the detection device to detect a target.

17. The sensor of claim 1, wherein, when the detection device has a negative resistance characteristic, the sensor detects one or more selected from the group consisting of a current and a voltage of the detection device to detect a target.

18. The sensor of claim 1, wherein, when the detection device does not have a negative resistance characteristic, the sensor detects any one or more selected from a current and a voltage output by the detection device to detect a target.

19. The sensor of claim 1, wherein, when the detection device does not have a negative resistance characteristic, current-voltage pairs output by the detection device form a line graph having a slope, wherein the slope is changed according to a concentration of a target and whether a target is present.

20. The sensor of claim 1, which controls the driving power provided to the actuator and the detection device such that an electrical signal output by the detection device is controllable to have a negative resistance characteristic or a positive resistance characteristic.
